# EUROPEAN PATENT SPECIFICATION

(11) **EP 0 434 836 B1**
(45) Date of publication and mention of the grant of the patent: **25.10.1995**
(21) Application number: 89909433.8
(22) Date of filing: 17.08.1989
(51) Int. Cl.: C07K 14/47, C07K 1/18, C07K 1/22, C12P 21/00

(54) **METHOD FOR PURIFYING TENASCIN**
VERFAHREN ZUR REINIGUNG VON TENASCIN
PROCEDE DE PURIFICATION DE TENASCINE

(30) Priority: 19.08.1988 JP 205902/88
(43) Date of publication of application: 03.07.1991
(73) Proprietor: RIKAGAKU KENKYUSHO, Wako-shi Saitama-ken 351-01 (JP); JAPAN IMMUNORESEARCH LABORATORIES CO., LTD., Gunma-ken 370 (JP); AMANO PHARMACEUTICAL CO., LTD., Nagoya-shi, Aichi-ken 460 (JP); NIPPON SHINYAKU COMPANY, LIMITED, Minami-ku Kyoto-shi Kyoto 601 (JP)
(72) Inventor: SAKAKURA, Teruyo Tsukuba Life Science Center,, Tsukuba-shi Ibaragi-ken 305 (JP); OIKE, Yasuteru Tsukuba Life Science Center,, Tsukuba-shi Ibaragi-ken 305 (JP); HIRAIWA, Hideki Tsukuba Life Science Center,, Tsukuba-shi Ibaragi-ken 305 (JP); KAWAKATSU, Hisaaki Tsukuba Life Science Center,, 3-chome Tsukuba-shi Ibaragi-ken 305 (JP)
(74) Representative: Werner, Hans-Karsten, Dr.Dipl.-Chem.
(86) International application number: JP8900839
(87) International publication number: WO9002140

(56) References cited:
- JP-A- 5 933 295
- JP-A-61 148 127

## Description

### BACKGROUND OF THE INVENTION

### FIELD OF THE INVENTION

The present invention relates to a process for purifying extracellular stromal components, and more specifically, a process for purifying the extracellular stromal component, tenascin.

### DESCRIPTION OF THE PRIOR ART

Tenascin is glycoprotein induced in the stromal tissue of a cell, which happened to be isolated in the course of an investigation into yielding a monoclonal antibody against type V collagen and was identified as an antigen defined by an antibody which has a staining ability distinguishable from that of type V collagen. Tenascin, one of the extracellular stromal components, has been found to have various biological activities which include, for example, a hemagglutination activity more potent than fibronectin, which is believed to result in a regulation for histogenesis (Oike et al., Seitai No Kagaku, Vol 39: pp.45-47, 1988), a non-covalent interaction with other biological molecules such as an interaction with fibronectin (Chiquet et al., Cell, Vol 53: pp.383-390, 1988), a possible interaction with proteoglycan (Chiquet et al., J. Cell Biol., Vol 98: pp.1926-1936, 1984). Moreover tenascin performs various physiological activities such as a calcium-dependent cell attachment activity (Bourdon et al., J. Cell Biol., Vol 105: p.138a) and a cell propagation enhancing activity after it is induced in the mesenchyme after the development of epithelial carcinoma (Chiquet-Ehrismann, R. et al., Cell., Vol 47: pp.131-139, 1986), which makes tenascin important in cell propagation or morphogenesis.

Although tenascin is such an important physiological substance, it is not human-tenascin but other species-tenascin that has been investigated and thus the physiological activities of human-tenascin have not been revealed sufficiently. A process for purifing tenascin, which has been used up to the present, is affected by affinity chromatography in which gel beads covalently bonded to monoclonal or polyclonal antibodies of a chicken are used. This process is not used widely because it requires a special operation, and could not be used for the purification of other species-tenascin, especially human-tenascin. Further it is difficult to obtain highly purified human-tenascin because the yield of human-tenascin which is produced by human-fibroblast under a culture condition is about 2 % on the basis of the weight of the fibronectin. On the other hand, M. A. Bourdon et al have recently found human glioma-mesenchymal extracellular matrix antigen (Human Glioma-Mesenchymal Extracellular Matrix Antigen, GMEM Antigen, Cancer Research, Vol 43 : pp.2796-2805, 1983) and identified it as human-tenascin (Bourdon et al, Journal of Biology, Vol 105 : p.138 a 1987). The process for purifying a human-tenascin requires an affinity chromatography prepared with antibodies which were obtained from mouse ascites containing a large amount of monoclonal antibodies produced by hybridomas to glioma U251 and then were purified. This purification process was unusual and has the disadvantage that any industrial application of it is very expensive.

If monoclonal antibodies against human-tenascin can be obtained with highly purified human-tenascin, it would be possible to apply the monoclonal antibodies for use in diagnostication and treatment of epithelial cancer and the like. Thus, a development of an industrially appliable process for purifying human-tenascin only by the usual biochemical techniques and a highly purified human-tenascin are desired.

Therefore, the object of the present invention is to provide a process for obtaining a high yield of highly purified human-tenascin by the usual biochemical techniques.

The inventors have found that the above problem is solved by a process comprising the steps of extracting tenascin from human fibroblast and so on, removing fibronectin and purifying the tenascin by an anion ionexchange resin for a biopolymer separation to accomplish the present invention.

The present invention provides a process for purifying tenascin characterized in that the process comprises the steps of removing fibronectin from an extracellular stroma extract containing tenascin so that the content ratio of the fibronectin is not more than 1% by weight on the basis of the total solute of the effluent by using affinity chromatography media containing gelatin covalently attached to a matrix and then purifying the tenascin by a basic anion exchange resin.

### DISCLOSURE OF THE INVENTION

A detailed explanation of the invention will be made hereinunder. As a source of tenascin used for the process for purifying tenascin according to the present invention, human fibroblast cells as well as human melanoma cells, human glioma cells, human chondrosarcoma cells and the like can be used. In addition, as a source of other species-tenascin than human-tenascin, mouse embryos, chicken embryos and the like can be used.

In the case where such a human fibroblast cell is used, the cell may be cultured in such a culture medium as fetal calf serum until the cells become confluent, and a mixture of proteins containing human-tenascin may then be extracted.

From these cells, a cell-surface extract can be extracted by a known method, for example, by a process involving the use of urea, guanidine hydrochloride, guanidine isothiocyanate and the like. Generally, urea may be used at a concentration of 1 - 8 M, preferably at 2 M, and guanidine hydrochloride or guanidine isothiocyanate may be used at a concentration of not less than 3 M. Other additives, for example, phenylmethylsulfonium fluoride (PMSF), may be used in addition to the above extracting agents.

In the case where such a human fibroblast cell is used, an extract can be extracted with such a solvent as 2 M urea/ phosphate buffered saline (PBS)/ 2 mM-PMSF from the cell wall of the cell.

Subsequently, a mixture of proteins containing human-tenascin can be precipitated with ammonium sulfate or the like. Ammonium sulfate may generally be used at a concentration of 60 - 80 % for 2 hours at 4°C to precipitate a mixture of proteins containing human-tenascin, collagen, proteoglycan, fibronectin and other unknown proteins.

Generally, the mixture of proteins contains fibronectin at about 300µ g /ml, equal to almost 50 times the content of tenascin, and thus further purification is required to remove fibronectin. The precipitated mixture of proteins is preferably applied to preliminary purification by gel filtration after being dissolved in the solution containing 2 M urea, preferably 2 M urea/ 0.15 M sodium chloride/ 50 mM Tris-HCl buffer (pH 8.0) at 4°C.

As a resin for the gel filtration there can be used such a resin as, for example, Sepharose CL2B, Sepharose CL4B and Sepharose CL6B (Pharmacia Co.), preferably Sepharose CL4B. Other resins such as Bio-gel 5m, Bio-gel 15m (Bio-Rad Co.), Sephacryl S500, Sephacryl S1000 (Pharmacia Co.) may be used.

Tenascin eluted from the column can be detected by measuring tenascin-dependent cell attachment activity (Methods in Enzymology, Vol 82: pp.803 - 831, 1982). Examples of cells useful for measuring the cell attachment activity include human melanoma cells, human fibroblast cells, human epithelial carcinoma cells and the like. Other methods, for example, enzyme linked immunoadsorbent assay (ELISA) using a polyclonal antibody against chicken-tenascin, can be used for identification. Fibronectin may be detected by measuring the immunoreactivity of the effluent against fibronectin (Archives of Biochemistry and Biophysics,pp. 399 - 406, 1981).

A preliminarily purified human-tenascin can be obtained by collecting the effluent fractions containing not less than 20%, preferably not less than 10%, of human-tenascin. The preliminarily purified human-tenascin generally contains about 50% of fibronectin and thus it is required that, after being concentrated and dialyzed in the usual manner, the purified human-tenascin be applied on a gelatin gel in order to remove the fibronectin.

The fraction collected is preferably concentrated by such a membrane as a Centri-flow membrane (Amicon Co.) at 2,500× g at 4°C and preferably dialyzed against a buffer containing 0.5 M urea, preferably 0.5 M urea/ 0.15 M sodium chloride/ 50 mM Tris-HCl (pH 7.4) for 12 hours at 4 °C.

Examples of gelatin gels include Gelatin-Sepharose (Pharmacia Co.), Immobilized Gelatin (Piearce Co.), Gelatin-Agarose (Sigma Co.) and the like, which are affinity chromatography media containing gelatin covalently attached to a matrix. By using the gelatin column having a high affinity to fibronectin, tenascin can elute into a run-through fraction to remove fibronectin so that the content ratio of the fibronectin is not more than 1% by weight on the basis of the total solute of the effluent. The fibronectin can be detected by the ELISA method using a polyclonal antibody against human-fibronectin, and the tenascin can be also detected by the ELISA method described above.

The eluted fractions obtained by the above method may be concentrated by an ordinary method and dialyzed against a solution containing 2 M urea, preferably against a solution of 2 M urea/ 50 mM Tris-HCl (pH 8.0), to give a solution containing tenascin in a high concentration.

The solution obtained generally contains 100µ g/ml of human tenascin, which is subsequently subjected to purification using an anion exchange resin for a biopolymer separation to obtain an extremely highly purified tenascin.

Examples of the purification process include a process using high performance liquid chromatography (HPLC) comprising an ion exchange column such as a DEAE-5PW column (Toso Co; Japan) which contains an ion exchange resin modified with a diethylaminoethyl (DEAE) group for the ion exchange group. Other ion exchange columns such as DEAE-Toyopal 650 (Toso Co; Japan), Mono-Q, DEAE-CL6B, DEAE-Sephacel (Pharmacia Co.), DEAE-Cellulofine (Seikagaku-Kogyo; Japan) and the like may be used. Among these ion exchangers, DEAE-5PW is preferably used. As an eluent for the exchanger there can be used a solution containing sodium chloride at a concentration of 0-1 M, preferably 0-0.4 M in urea at a concentraion of 1 - 8 M, preferably 2 M.

In the case where a DEAE-5PW column is used with an eluent of the linear ascending gradient of 2 M urea/ 0 - 0.4 M sodium chloride for the ion exchange HPLC, the tenascin can generally be eluted with 0.2 - 0.3 M sodium chloride, preferably about 0.25 M sodium chloride. The eluted human-tenascin may be detected by measuring ultra violet absorption or cell attachment activity specific to tenascin.

By the purification processes hereinbefore mentioned, tenascin with about 100% purity can be obtained. The human-tenascin purified by the process according to the present invention exhibited distinct protein bands of 250K and 200K in SDS-PAGE analysis, which bands were specific for human-tenascin. The yield of tenascin by the process for purification according to the present invention is generally not less than 80%.

A crude tenascin fraction can also be obtained from a culture medium by using ammonium sulfate at a concentraion of 30 - 35%, preferably 33.3%. The same purification process may be applied to the crude tenascin fraction to obtain the purified tenascin.

### BRIEF DESCRIPTION OF THE DRAWINGS

FIG. 1 shows the elution profile of human-tenascin through a Sepharose CL-4B column. The human-tenascin was extracted from human fibroblast cells.

FIG. 2 and FIG. 3 each shows a high performance liquid chromatogram of a fraction which passed through a column containing gelatin. A DEAE-5PW column was used for the purification and the purified human-tenascin was detected by ultra violet absorption (FIG. 2) and cell attachment activity(FIG. 3).

FIG. 4 shows the elution profile of human-tenascin through a Sephrose CL-4B column. The human-tenascin was extracted from the culture medium of human fibroblasts.

FIG. 5 and FIG. 6 each shows a high performance liquid chromatogram of a fraction which passed through a column containing gelatin. A DEAE-5PW column was used for the purification and the purified human-tenascin was detected by ultra violet absorption (FIG. 5) and cell attachment activity(FIG. 6).

FIG. 7 and FIG. 8 each shows a high performance liquid chromatogram of a fraction which passed through a column containing gelatin. A DEAE-5PW column was used for the purification and the purified human-tenascin was detected by ultra violet absorption. The human-tenascin was extracted from human melanoma cells(FIG. 7) and human chondrosarcoma cells(FIG. 8).

### THE BEST MODE OF THE INVENTION

The best mode of the present invention will be further explained in detail with reference to the following examples. The present invention is not limited to the following examples.

### Example 1

Human fibroblast cells were plated on one hundred roller bottles each containing a culture medium of Dulbecco's modified Eagle medium/ Ham F-12/ 10% fetal calf serum, and the cells were cultured at 37°C . After the cells became confluent, 10*ml* of 2M urea/Ham F-12/2mM phenylmethylsulfonium fluoride (PMSF) was added to a cell layer of each bottle and a mixture of proteins containing human-tenascin was extracted while rotating the roller bottles (1 rpm) at 37 °C for two hours. After cooling the solution which contains the extract to 4 °C , ammonium sulfate was added to the solution at 70% by weight saturation. After gentle mixing, the resulting solution was left to stand at 4°C for 2 hours to precipitate the mixture of proteins containing human-tenascin as well as collagen, proteoglycan, fibronectin and other unknown proteins.

The mixture of proteins was collected by centrifugation at 20,000 rpm for 1 hour at 4 °C and the supernatant was removed by decantation. The precipitate was then dissolved in 200 ml of 2 M urea/0.15 M sodium chloride/ 50 mM Tris-HCl (pH 8.0). The total amount of the protein contained in the solution was 100 mg (measured by BCA protein assay, Piearce Co.).

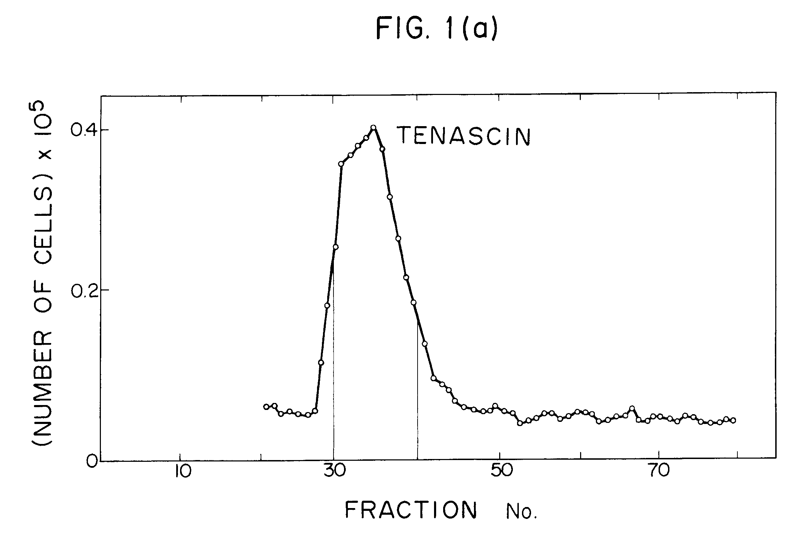

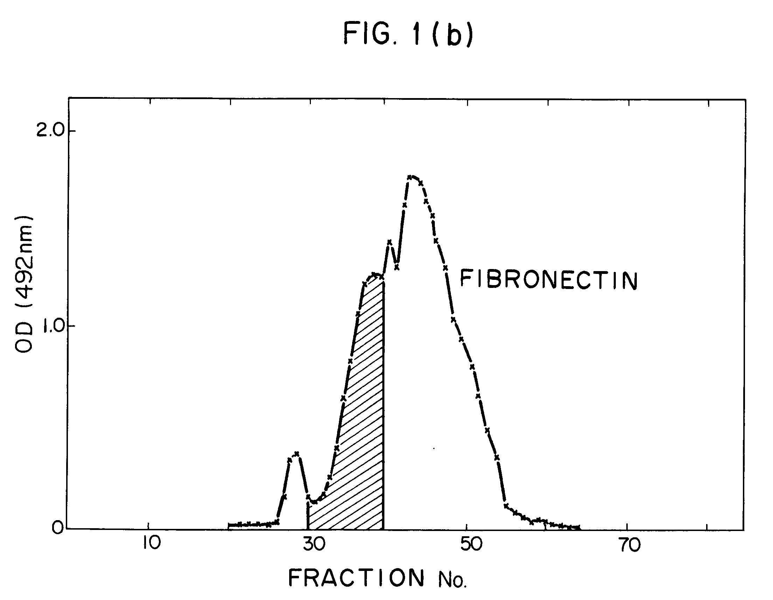

10 ml of the solution was applied on a Sepharose CL4B column (bed volume of about 500 ml, Pharmacia Co.) and human-tenascin was eluted at 15°C to separate it from fibronectin with an eluent of 2 M urea/0.15 M sodium chloride/ 50 mM Tris-HCl (pH 8.0) (FIG. 1).

Tenascin dependent cell attachment activity was measured as follows: a 200µ l sample from each fraction was added into the well of an ELISA plate (Libro, 76-203-05, Titertek Co.) and then 20µ l of 50 mM sodium carbonate buffer (pH 9.6) was added into the well and coated for 12 hours at 4°C . After the well was washed thrice with PBS/ 0.5% bovine serum albumin (BSA), 200µ l of 5% anti-fibronectin antibody (Cappel Co.) in PBS/ 0.5% BSA was added into the well. After the plate was incubated for 2 hours at 37°C , the well was washed with PBS/ 0.5% BSA.

In the meantime, human fibroblasts, which had been maintained until the cells became confluent in a culture medium of Dulbecco's modified Eagle medium/ Ham F-12/ 10% fetal calf serum, were treated with 0.1 mg/ml solution of trypsin free from EDTA (Type III, Sigma Chemical Co.) for 1 hour at 37°C to suspend the cells. A cell suspension of 1 × 10⁶ cell/ml in PBS/ 0.5 mg soybean trypsin inhibitor (Sigma Co.) was prepared. 100µ l of the cell suspension was added into the well of the microtiter plate obtained above and the plate was incubated for 1 hour at 37°C . After being washed once with PBS/ 0.5% BSA, the well was treated with 3% paraformaldehyde to immobilize the cells adhering to the surfaces of the well. The cells were stained with 1%-toluidine blue (Merck)/3% paraformaldehyde, and the number of cells was determined by microscopic measurement.

The amount of fibronectin was determined by the following method which includes the measurement of antigenecity against fibronectin. 20 µ l of a sample from each fraction was added into the well of an ELISA plate (3912, Pharcom Co.). 180 µ l of 50 mM sodium carbonate buffer (pH 9.6) was added into the well and then the plate was incubated for 12 hours at 4°C . After the well was washed thrice with PBS/ 0.5%(W/W) Tween 20/ 0.5%(W/W) BSA, 200 µ l of rabbit anti-fibronectin antibody (0.4% , Cappel Co.) was added into the well and then the plate was incubated for 1 hour at 20 °C . The antibody solution was discarded and subsequently the well was washed with PBS/ 0.5%(W/W) Tween 20/ 0.5%(W/W) BSA and then supplied with the solution of anti-goat antibody conjugated with peroxidase (0.4%, Cappel Co., Ltd). After the plate was incubated for an additional 1 hour, the well was washed with PBS/ 0.5%(W/W) Tween 20/ 0.5%(W/W) BSA. The peroxidase activity of each well was measured spectrophotometrically at 492 nm with a substrate of ortho-phenylenediamine hydrochloride.

The above process was repeated 10 times. FIG. 1 shows that tenascin was eluted at a Kd value of 0.11 and was incompletely separated from fibronectin by Sepharose column. Fractions No. 30 - 40 (each of 5 ml) in FIG. 1 were gathered to obtain 500 ml of the solution containing tenascin in a high concentraion. The total amount of protein was 2.6 mg. After being concentrated to 50*ml* by centrifugation at 2500× g using a Centriflow membrane (Amicon Co.) at 4 °C , the solution was dialyzed against a 1-liter solution of 0.5 M urea/ 0.15 M sodium chloride/ 50 mM Tris-HCl (pH 7.4) for 12 hours at 4 °C.

60*ml* of the solution obtained was then applied on a 10 ml bed volume of gelatin column (Gelain Sepharose, Pharmacia Co.). Fibronectin contained in the solution was specifically adsorbed on the resin and purified human-tenascin was eluted to give 70ml of the tenascin solution which was free from fibronectin.

The solution which contains the effluent was concentrated to 20 ml at 2500× g using Centriflow membrane (Amicon Co.) at 4°C and then dialyzed for 12 hours at 4°C against 1 liter of 0.5 M urea/ 50 mM Tris-HCl (pH 8.0).

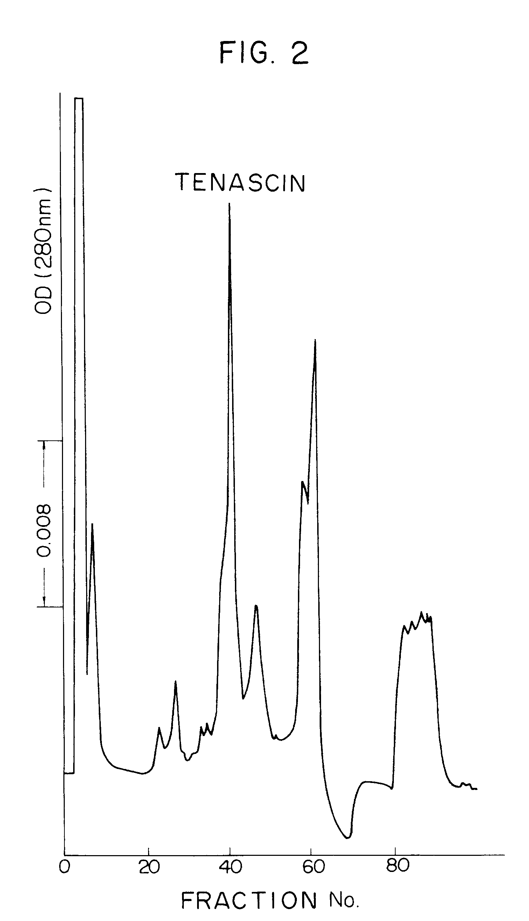

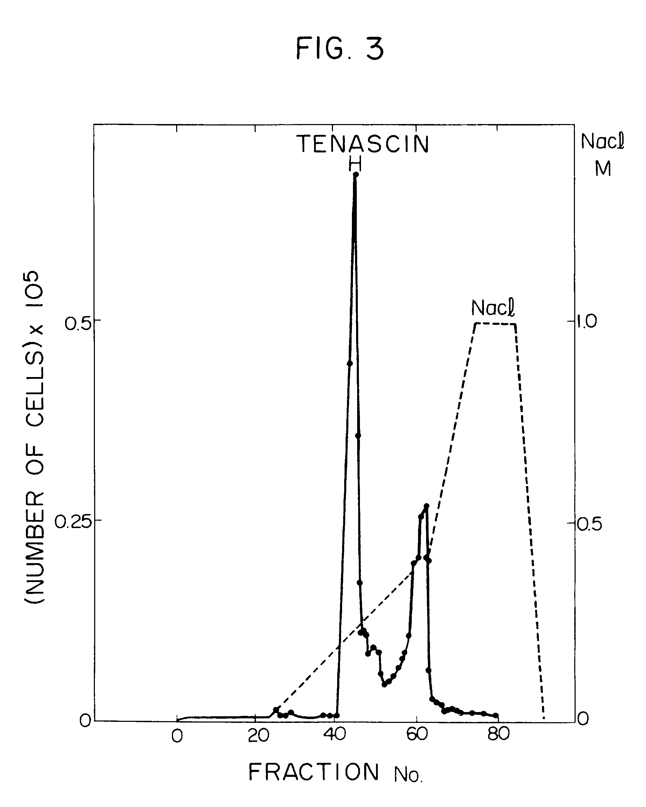

The solution obtained was purified by HPLC with a DEAE-5PW column (7.5 mm × 50 mm, Toso Co; Japan). A 1 ml sample was injected in the column and eluted with a linear ascending gradient eluent of 0 - 0.4 M sodium chloride in 2M urea at the flow rate of 0.5*ml* /min with a column pressure of 15 Kg/ cm² while the column temperature was maintained at 15°C . Desired tenascin was eluted at 2M urea/ 0.25 M sodium chloride in a retention time of 42 - 45 minutes, which tenascin was detected by measuring the absorbance of the effluent at 280 nm and the tenascin dependent cell attachment activity mentioned above. The results are summarized in FIG. 2 and FIG. 3. These figures show that the purified tenascin was eluted with an intensive single peak.

A total amount of 200 µ g of pure human-tenascin was obtained from 100 roller bottles (about 200× 10⁸ cells).

5 µ g of the human-tenascin obtained above was reduced for 30 minutes at 70 °C with 1% 2-mercaptoethanol/ 2% SDS/ 50 mM Tris-HCl (pH 8.0) and applied on SDS-polyacrylamide gradient gel (4% - 20%) for analysis. Two bands of 250K and 200K identified with human-tenascin were detected without detecting any additional band, thus proving the obtained human-tenascin to be of about 100% purity.

### Example 2

Human fibroblast cells were plated on one hundred roller bottles (850 cm³) each containing 110 *ml* culture medium of Dulbecco's modified Eagle medium/ Ham F-12/ 10% fetal calf serum, and the cells were cultured at 37°C with rotation of 1.1 rpm. After the cells became confluent, the culture medium was replaced with a fresh medium every 5 days. After cooling 100ℓ of the medium to 4°C , ammonium sulfate was added to the medium at 33% by weight saturation. After gentle mixing, the resulting solution was left to stand at 4°C for 120 minutes to precipitate the mixture of proteins containing human-tenascin as well as collagen, proteoglycan, fibronectin and other proteins.

The mixture of proteins was collected by centrifugation at 20,000 × g for 60 minures at 4°C and the supernatant was removed by decantation. The precipitate was then dissolved in 750 ml of 2 M urea/0.15 M sodium chloride/ 50 mM Tris-HCl (pH 8.0) at 4 °C . The total amount of the protein contained in the solution was 3.9 g (measured by BCA protein assay, Piearce Co.).

25 ml of the solution was applied on a Sepharose CL6B column (bed volume of about 500 ml, Pharmacia Co.) and human-tenascin was eluted at 15°C to separate it from fibronectin with an eluent of 2 M urea/0.15 M sodium chloride/ 50 mM Tris-HCl (pH 8.0).

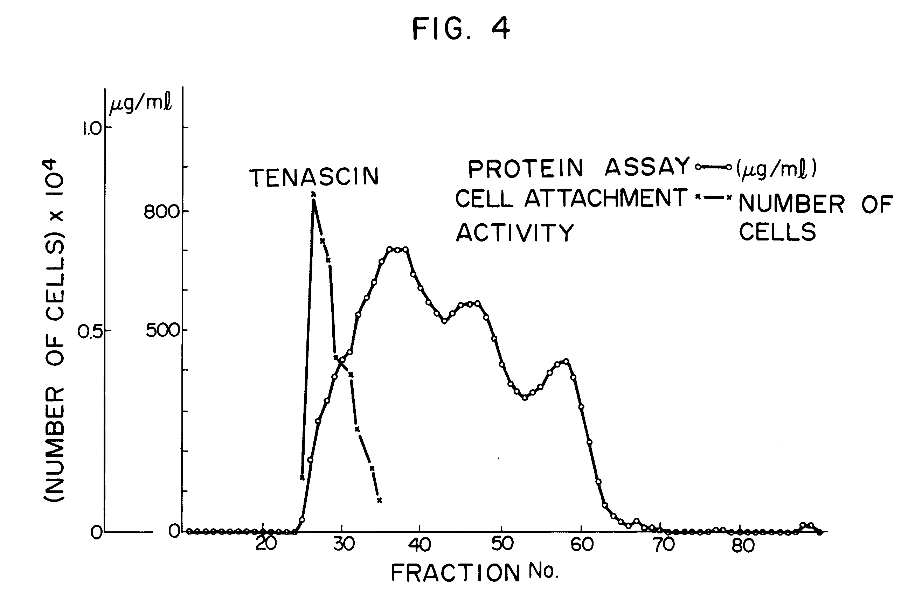

Tenascin dependent cell attachment activity was measured by the same method described in Example 1. Fibronectin was determined by the same method described in Example 1, which method includes the measurement of antigenecity against fibronectin. The above process was repeated 30 times. Fig. 4 shows the elution profiles of the tenascin and the fibronectin. Fig. 4 shows that human-tenascin was eluted at Kd value of zero and was incompletely separated from fibronectin by Sepharose column. Fractions No. 25 - 30 (each of 5 ml) in FIG. 3 were gathered to obtain 900 ml of the solution containing human-tenascin in a high concentraion. The total amount of protein was 90.0 mg.

After being concentrated to 100 *ml* by centrifugation at 2500× g using a Centriflow membrane (Amicon Co.) at 4 °C , the solution was dialyzed against a 2-liter solution of 0.5 M urea/ 0.15 M sodium chloride/ 50 mM Tris-HCl (pH 7.4) for 12 hours at 4 °C

120 *ml* of the solution obtained was then applied on a 30 ml bed volume of gelatin column (Gelain Sepharose, Pharmacia Co., Ltd) to specifically adsorb fibronectin on the resin. Human-tenascin was eluted in a run-through fraction to give 220ml of the tenascin solution which was free from fibronectin.

The solution which contains the effluent was concentrated to 40 ml at 2500× g using Centriflow membrane (Amicon Co.) at 4°C and then dialyzed for 12 hours at 4°C against 2 liters of 2 M urea/ 50 mM Tris-HCl (pH 8.0).

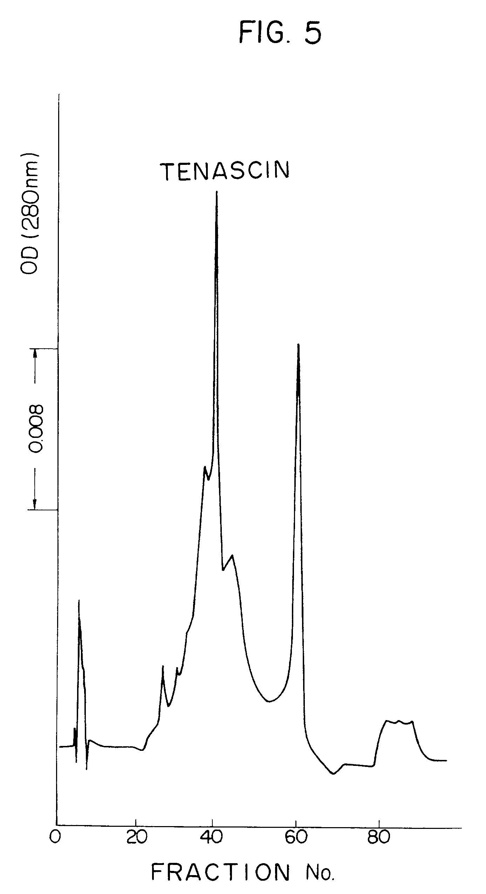

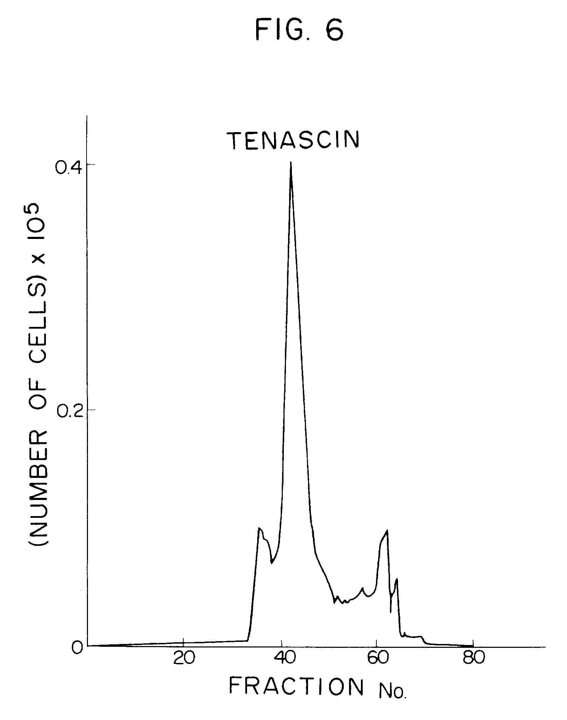

The solution obtained was purified by HPLC with a DEAE-5PW column (7.5 mm × 50 mm, Toso Co; Japan). A 1 ml sample was injected in the column and eluted with a linear ascending gradient eluent of 0 - 0.4 M sodium chloride in 2M urea at the flow rate of 0.5*ml*/min with a column pressure of 15 Kg/ cm² while the column temperature was maintained at 15°C . The desired human-tenascin was eluted at 2M urea/ 0.25 M sodium chloride in a retention time of 42 - 45 minutes, which human-tenascin was detected by measuring the absorbance of the effluent at 280 nm and the tenascin dependent cell attachment activity described in Example 1. The results are summarized in Fig. 5 and Fig. 6. These figures show that the purified tenascin was eluted with an intensive single peak. A total amount of 6.6 mg of pure human-tenascin was obtained from 100 roller bottles, 100 ℓ of the medium (about 200× 10⁸ cells).

5 µ g of the human-tenascin obtained above was reduced for 3 minutes at 100 °C with 1% 2-mercaptoethanol/ 2% SDS/ 50 mM Tris-HCl (pH 6.8) and applied on SDS-PAGE (4% - 20%) for analysis. Two bands of 250K and 200K identified with human tenascin were detected but no additional band was detected, thus proving the obtained human-tenascin to be of about 100% purity.

### Example 3

Human melanoma cells (M-14) were injected into the hypoderm of a nude mouse at 2× 10⁶ cell/mouse and cancerous tissues were delivered therefrom after about 1 month.

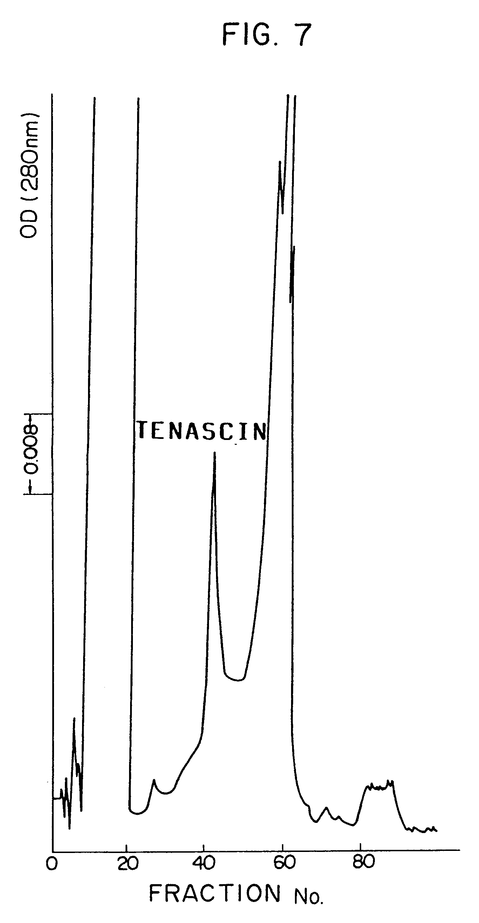

5 g of the delivered tissues was ground and 45*ml* of 2 M urea/50 mM Tris-HCl (pH 7.4)/0.15 M sodium chloride/2 mM PMSF was added to the ground tissues, followed by stirring at 4°C for 12 hours to yield 310 mg of a fraction containing tenascin by the method described in Example 2. An eluation condition is the same as that of Example 2. The void fraction (9 mg ) was purified by DEAE-5PW after removing fibronectin by the same procedure as that of Example 1 to obtain 700µ g of tenascin(FIG. 7). SDS-PAGE in which the same procedure as Example 1 was repeated revealed that the obtained tenascin had about 100 % purity.

### Example 4

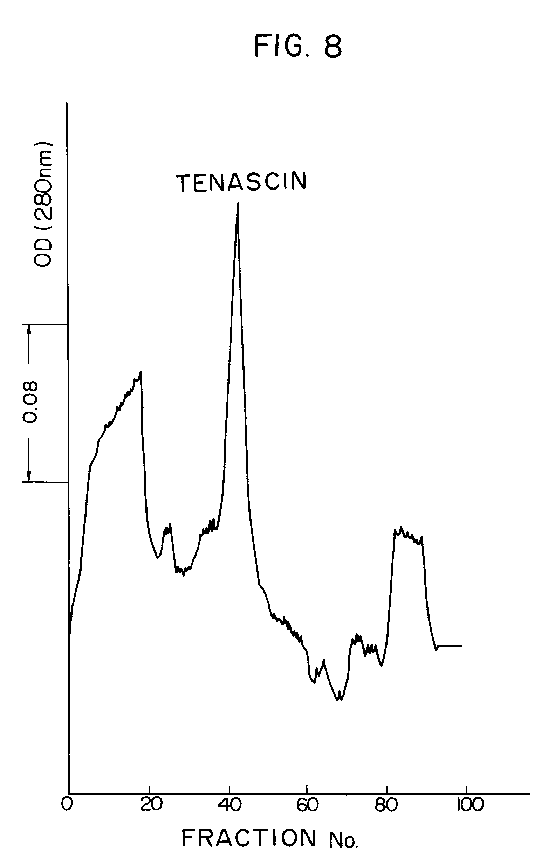

27*ml* of 2 M urea/50 mM Tris-HCl (pH 7.4)/0.15 M sodium chloride/2 mM PMSF was added to 3 g of human-chondrosarcoma tissues, followed by stirring at 4 °C for 12 hours to yield 30 mg of a fraction containing tenascin. An eluation condition of Sepharose CL-6B is the same as that of Example 2. The void fraction (1 mg ) was purified by DEAE-5PW after removing fibronectin by the same procedure as that of Example 1 to obtain 200µ g of tenascin(FIG. 8). SDS-PAGE in which the same procedure as Example 1 was repeated revealed that the obtained tenascin had about 100 % purity.

### Example 5

13.5*ml* of 2 M urea/50 mM Tris-HCl (pH 8.0)/0.15 M sodium chloride/2 mM PMSF was added to 400 mouse nine day old embryos (1.5 g), followed by stirring at 4°C for 12 hours to yield 15 mg of a fraction containing mouse-tenascin. An eluation condition of Sepharose CL-6B is the same as that of Example 2. The void fraction (500 µ g was purified by DEAE-5PW after removing fibronectin (400µ g) by the same procedure as that of Example 1 to obtain 100 µ g of tenascin. An elution pattern was nearly the same as that of Example 1. SDS-PAGE in which the same procedure as Example 1 was repeated revealed that the obtained tenascin had about 100% purity.

### Example 6

9 *ml* of 2 M urea/50 mM Tris-HCl (pH 8.0)/0.15 M sodium chloride/2 mM PMSF was added to 300 chicken embryos (Hamburger-Hamilton stage 23.5), followed by stirring at 4 °C for 12 hours to yield 10 mg of a fraction containing mouse-tenascin. An elution condition of Sepharose CL-6B is the same as that of Example 2. The void fraction (300µ g ) was purified by DEAE-5PW after removing fibronectin (250µ g) by the same procedure as that of Example 1 to obtain 60µ g of tenascin. An elution profile was nearly the same as that of Example 1. SDS-PAGE in which the same procedure as Example 1 was repeated revealed that the obtained tenascin had about 100% purity.

### INDUSTRIAL APPLICABILITY

The process for purifying tenascin according to the present invention can provide a pure biochemical technique which can be applied industrially to obtain a high yield of extremely highly purified tenascin, which can be used in producing bioactive substances such as a monoclonal antibody against tenascin and the like, which are useful, for example, for the diagnosis and treatment of cancer.

## Claims

1. A process for purifying tenascin characterized in that the process comprises the steps of removing fibronectin from extracellular stromal extracted materials containing tenascin so that the content ratio of the fibronectin is not more than 1 % by weight on the basis of the total solute of the effluent by using affinity chromatography media containing gelatin covalently attached to a matrix and then applying a run-through fraction on a basic anion exchange resin to purify the tenascin.

2. A process according to claim 1, wherein the extracellular stromal extracted materials are derived from a cell selected from the group consisting of human-fibroblast cells, human-melanoma cells, human-glioma cells, and human-chondrosarcoma cells.

3. A process according to claim 1, wherein the extracellular stromal extracted materials are eluted with an extracting agent selected from the group consisting of urea, guanidine hydrochloride and guanidine isothiocyanate.

4. A process according to claim 2, wherein the extracellular stromal extracted materials are obtained from a cell culture medium.

5. A process according to claim 1, wherein the basic anion exchage resin is a resin modified with diethylaminoethyl groups.

6. A process according to claim 1, wherein the purification by the basic anion exchange resin is affected by using not more than 1 M sodium chloride in 1-8 M urea.

7. A process according to claim 1, wherein the purification by the basic anion exchange resin is affected by using not more than 0.4 M sodium chloride in 2 M urea.

8. A process according to claim 1, wherein the purification by the basic anion exchange resin is affected by using a linear ascending gradient of 0-0.4M sodium chloride in 2M urea.

9. A process according to claim 8, wherein the purification by the basic anion exchange resin is affected by a high performance liquid chromatography.

10. A process according to claim 1, wherein tenascin is detected by tenascin dependent cell attachment activity.

## Patentansprüche

1. Verfahren zur Reinigung von Tenascin, dadurch gekennzeichnet, daß das Verfahren die Stufen des Entfernens von Fibronectin aus extrazellulären, extrahierten Stroma-Materialien, die Tenascin in einem derartigen Maß enthalten, daß das Verhältnis des Gehalts an Fibronectin nicht größer als 1 Gew.-% bezogen auf den gesamten gelösten Stoff des Ausflusses ist, durch Anwendung von Affinitätschromatographie-Medien, die Gelatine kovalent an eine Matrix gebunden enthalten, und anschließendes Anwenden einer Durchlauf-Fraktion auf einem basischen Anionenaustauscherharz zur Reinigung des Tenascins, umfaßt.

2. Verfahren gemäß Anspruch 1, worin die extrazellulären, extrahierten Stroma-Materialien aus einer Zelle stammen, die aus der Gruppe, bestehend aus Human-Fibroblastzellen, Human-Melanomzellen, Human-Gliomzellen und Human-Chondrosarkomzellen, ausgewählt ist.

3. Verfahren gemäß Anspruch 1, worin die extrazellulären, extrahierten Stroma-Materialien mit einem Extraktionsmittel extrahiert werden, das aus der Gruppe, bestehend aus Harnstoff, Guanidin-Hydrochlorid und Guanidin-Isothiocyanat ausgewählt ist.

4. Verfahren gemäß Anspruch 2, worin die extrazellulären, extrahierten Stroma-Materialien aus einem Zellkulturmedium erhalten werden.

5. Verfahren gemäß Anspruch 1, worin das basische anionische Austauscherharz ein Harz ist, das mit Diethylaminoethyl-Gruppen modifiziert ist.

6. Verfahren gemäß Anspruch 1, worin die Reinigung durch das basische anionische Austauscherharz unter Anwendung von nicht mehr als 1 M Natriumchlorid in 1-8 M Harnstoff erfolgt.

7. Verfahren gemäß Anspruch 1, worin die Reinigung durch das basische anionische Austauscherharz unter Anwendung von nicht mehr als 0,4 M Natriumchlorid in 2 M Harnstoff erfolgt.

8. Verfahren gemäß Anspruch 1, worin die Reinigung durch das basische anionische Austauscherharz unter Anwendung eines linear aufsteigenden Gradienten von 0-0,4 M Natriumchlorid in 2 M Harnstoff erfolgt.

9. Verfahren gemäß Anspruch 8, worin die Reinigung durch das basische anionische Austauscherharz unter Anwendung einer Hochleistungs-Flüssigchromatographie erfolgt.

10. Verfahren gemäß Anspruch 1, worin das Tenascin durch Tenascin-abhängige Zellbindungsaktivität entdeckt wird.

## Revendications

1. Procédé de purification de la ténascine caractérisé en ce que le procédé comprend les étapes consistant à enlever la fibronectine de substances extracellulaires de stroma extraites contenant de la ténascine, de sorte que le rapport de quantité de la fibronectine n'est pas supérieur à 1% en masse par rapport au soluté total de l'effluent, en utilisant des milieux de chromatographie par affinité contenant de la gélatine liée de façon covalente à une matrice, puis à appliquer une fraction passante sur une résine échangeuse d'anions basiques pour purifier la ténascine.

2. Procédé selon la revendication 1, dans lequel les substances extracellulaires de stroma extraites proviennent d'une cellule choisie dans le groupe constitué de cellules de fibroblastes humains, de cellules de mélanomes humains, de cellules de gliomes humains et de cellules de chondrosarcomes humains.

3. Procédé selon la revendication 1, dans lequel les substances extracellulaires de stroma extraites sont éluées avec un agent d'extraction choisi dans le groupe constitué de l'urée, du chlorhydrate de guanidine et de l'isothiocyanate de guanidine.

4. Procédé selon la revendication 2, dans lequel les substances extracellulaires de stroma extraites sont obtenues à partir d'un milieu de culture cellulaire.

5. Procédé selon la revendication 1, dans lequel la résine échangeuse d'anions basiques est une résine modifiée par des groupements diéthylaminoéthyle.

6. Procédé selon la revendication 1, dans lequel on réalise la purification par la résine échangeuse d'anions basiques en n'utilisant pas plus de 1 M de chlorure de sodium dans 1 à 8 M d'urée.

7. Procédé selon la revendication 1, dans lequel on réalise la purification par la résine échangeuse d'anions basiques en n'utilisant pas plus de 0,4 M de chlorure de sodium dans 2 M d'urée.

8. Procédé selon la revendication 1, dans lequel on réalise la purification par la résine échangeuse d'anions basiques en utilisant un gradient linéaire ascendant de 0 à 0,4 M de chlorure de sodium dans 2 M d'urée.

9. Procédé selon la revendication 8, dans lequel on réalise la purification par la résine échangeuse d'anions basiques par une chromatographie liquide haute pression.

10. Procédé selon la revendication 1, dans lequel la ténascine est détectée par l'activité de fixation de cellule dépendante de la ténascine.
